# EUROPEAN PATENT SPECIFICATION

(11) **EP 3 135 191 B1**
(45) Date of publication and mention of the grant of the patent: **29.08.2018**
(21) Application number: 16186481.4
(22) Date of filing: 31.08.2016
(51) Int. Cl.: A61B 5/00

(54) **ELECTRODE HOLDING ARRANGEMENT AND MANUFACTURING METHOD THEREOF**
ELEKTRODENHALTEANORDNUNG UND HERSTELLUNGSVERFAHREN DAFÜR
AGENCEMENT DE SUPPORT D'ÉLECTRODE ET SON PROCÉDÉ DE FABRICATION

(30) Priority: 31.08.2015 EP 15183098
(43) Date of publication of application: 01.03.2017
(73) Proprietor: Stichting IMEC Nederland, 5656 AE Eindhoven (NL)
(72) Inventor: Cramer, Pieter, 3001 Leuven (BE); Groenendaal, Bas, 3001 Leuven (BE)
(74) Representative: Patent Department IMEC

(56) References cited:
- WO-A1-91/04870
- GB-A- 2 219 187
- US-A1- 2008 114 232
- US-A1- 2011 041 851
- US-A1- 2013 211 223
- Dream 3d: "3D Printing A NinjaFlex Strap On The Ultimaker 2 | Cool 3D Prints | Dream 3D", Youtube, 21 August 2015 (2015-08-21), XP054977065, Retrieved from the Internet: URL:https://www.youtube.com/watch?v=5f6Bjm Ved1M [retrieved on 2017-01-16]

## Description

### Technical Field

The present invention relates generally to the field of biosignal acquisition or stimulation devices and systems and more specifically to an electrode holding arrangement for such applications in which an electrode for biosignal acquisition or stimulation shall be pressed towards a certain body tissue surface in order to make a stable and reliable electrical connection.

### Background

Monitoring of biosignals, such as for example and not limited to electrocardiogram (ECG), electroencephalogram (EEG) or electromyogram (EMG) signals, and the use of electrostimulators for pain relief or injury recovery, is a highly relevant topic in personal healthcare. An important challenge in some application environments, for example when using dry electrodes, is to achieve a stable and reliable electrode-skin contact interface for improving the electrical signal quality for sensing or stimulation purposes.

Some of such arrangements are known in the art, in which the electrodes are held and pressed, for example, towards the wrist, chest or scalp of a subject. One of such examples is patent application US 2013/0066184 A1, which describes a sensor headset with a contoured electrode that allows stable skin/head contact. US patent 6,574,513 B1 also describes a mounting device for positioning and holding wrapped electrodes on a scalp. Another example of an arrangement for holding electrodes on the head of a person is described in US patent 5,800,351. Patent application 2011/0041851A1 describes a device for detecting electrical potentials in the forehead area of a patient that has a transverse member adapted to rest against the forehead of the patient, a vertical member extending crosswise to the transverse member and a mask on the vertical member. Patent application US 2013/211223A1 describes an electrocardiograph electrode comprising one or more contact members provided with a plurality of contact pads suitable to establish an electrical contact with the body of a patient, wherein the contact members and/or contact pads are restrained to a supporting member of the electrode by way of a plurality of elastic members.

### Summary

The present description provides for a new electrode holding arrangement with improved capabilities compared to the state of the art solutions.

The scope of the invention is defined by the claims.

According to an exemplary embodiment, there is provided an electrode holding arrangement for biosignal acquisition or stimulation, comprising: a piece of flexible material adapted for being fixed to a certain body part; at least one electrode spring element created within the piece of flexible material by cutting out a certain geometric profile from said flexible material, and is configured for, when the piece of flexible material is fixed to the body part and an electrode is attached to the at least one electrode spring element and is positioned on a body tissue surface so that it generates a force that makes the electrode spring element protrude outside the horizontal plane of the piece of flexible material, generate a force that presses the electrode against the body tissue surface.

According to an exemplary embodiment, the electrode holding arrangement comprises one or more electrode spring elements that can be created by simply cutting away a certain profile of material from a piece of flexible material, which may be for example a single sheet of plastic that is also cut in a form that adapts to the required electrode applications (e.g. in the form of a headset, head, chest or wrist band or a patch). Therefore, the electrode holding arrangement may be advantageously manufactured from one sheet of flexible material, such as for example a flat sheet of plastic, using conventional cutting techniques, such as laser cutting, water jets, scissors or 3-D printing, which makes it very easy and cheap to manufacture. Furthermore, such electrode holding arrangement is compact and comprises less moving parts which, among other advantages, reduces complexity of manufacturing, assembly and mounting time. Since it is compact and can be made with lightweight material, the negative effects of motion artifacts are also advantageously reduced. Manufacturing of the electrode spring elements requires only one operation and is therefore cost efficient for any quantity, which allows for individual customization, for example, the electrode spring elements can be easily manufactured to obtain a certain desired constant and/or well controlled force or pressure applied on the electrodes: such force can be very efficiently controlled, since the electrode will tend to have a constant displacement once the arrangement is adjusted and the amount of force can be easily adapted and optimized by changing the flexible material properties and/or the geometry (the cutting profile pattern) of the electrode spring elements. It is also advantageous that the electrode spring element may be easily designed such that the direction of compression of the electrode is perpendicular to the body tissue surface, which improves the electrical signal quality. This is particularly advantageous for the use with dry electrodes and for applications that require that those electrodes are pressed to the body tissue for long periods of time without interfering with normal daily activities. According to an exemplary embodiment, the electrode holding arrangement is especially suited for the manufacture of EEG headsets, since the flexible material sheet from which the electrode spring element is made, can constitute the base part of the EEG cap to which the electronic components may be later applied. Furthermore, the electrode position, deflection and pressure can be easily adapted to different head sizes and shapes.

According to an exemplary embodiment, the at least one electrode spring element comprises at least: a first and a second connection arrangement connected to a middle segment piece; and wherein the at least first and second connection arrangement connects a different portion of the middle segment piece to a different anchor point of the piece of flexible material.

According to an exemplary embodiment, the at least first and second connection arrangement comprise at least one extendable element. According to an exemplary embodiment, the extendable element comprises a segment with a curved or meander shape. According to another exemplary embodiment, the extendable element comprises connected U-shaped segments. The connected U-shaped segments may have different lengths. According to another exemplary embodiment, the electrode holding arrangement comprises 3 or 4 connection arrangements.

According to an exemplary embodiment, the middle segment piece may have an opening for fixing an electrode to the electrode spring element. According to another exemplary embodiment, the electrode spring element further comprises an electrode holder.

According to an exemplary embodiment, the piece of flexible material is made of a plastic, cardboard or a plastic-like material, e.g. a material with similar performance as a plastic.

According to an exemplary embodiment, the piece of flexible material may be cut in a form that fits a certain body part.

According to an exemplary embodiment, the electrode holding arrangement further comprises fixing and/or fitting means to secure the piece of flexible material to a certain body part.

According to an exemplary embodiment, the electrode holding arrangement according to embodiments may be configured and used as a headset, headband, wristband or body arrangement for biosignal acquisition or stimulation.

The description also relates to a wearable device or garment comprising an electrode holding arrangement according to embodiments described in the claims.

The description also relates to a method for manufacturing an electrode holding arrangement comprising: cutting a piece of flexible material into a form that fits a certain body part; cutting out a certain geometric profile from said flexible material in order to create at least one electrode spring element within said piece of flexible material and such that the at least one electrode spring element is configured for, when the piece of flexible material is fixed to a body part and an electrode is attached to the at least one electrode spring element and is positioned on a body tissue surface so that it generates a force that makes the electrode spring element protrude outside the horizontal plane of the plastic piece of flexible material, generating a force that presses the electrode against the body tissue surface.

Certain objects and advantages of various new and inventive aspects have been described above. It is to be understood that not necessarily all such objects or advantages may be achieved in accordance with any particular embodiment of the present invention as described in the claims. Those skilled in the art will recognize that the solution as described in the claims may be embodied or carried out in a manner that achieves or optimizes one advantage or group of advantages without necessarily achieving other objects or advantages.

### Brief description of the drawings

The above and other aspects of the electrode holding arrangement and manufacturing method thereof will be shown and explained with reference to the non-restrictive example embodiments described hereinafter.
**Figures 1A** and **1B** show a top and side view of a general schematic representation of an electrode holding arrangement comprising a piece of flexible material and an electrode spring element according to a first exemplary embodiment.
**Figures 2A** and **2B** show a side view of the electrode holding arrangement of figure 1 comprising an electrode and with the electrode spring element in neutral state and in operation according to exemplary embodiments.
**Figure 3** shows a perspective view of the electrode holding arrangement of figure 2B when in operation according to an exemplary embodiment.
**Figure 4** shows another top view of a general schematic representation of an electrode holding arrangement according to a second exemplary embodiment.
**Figure 5** shows a top view of an exemplary implementation of an electrode spring element according to a third embodiment.
**Figures 6A** and **6B** show a side view of the electrode holding arrangement of figure 4 comprising and electrode and with the electrode spring element in neutral state and in operation according to exemplary embodiments.
**Figure 7** shows a top view of an exemplary implementation of an electrode spring element according to a fourth embodiment.
**Figure 8** shows a top view of another electrode spring element according to a fifth exemplary embodiment.
**Figure 9** shows a top view of another electrode spring element according to a sixth exemplary embodiment.
**Figure 10** shows a top view of another electrode spring element according to a seventh exemplary embodiment.
**Figures 11A** and **11B** show a top view of further exemplary embodiments of electrode spring elements.
**Figure 12** shows an exemplary electrode holding arrangement in the form of a headset device.
**Figure 13** shows an exemplary electrode holding arrangement in the form of a headband .
**Figure 14** shows an exemplary electrode holding arrangement in the form of a wristband .

### Detailed description

In the following, in the description of exemplary embodiments, various features may be grouped together in a single embodiment, figure, or description thereof for the purpose of streamlining the disclosure and aiding in the understanding of one or more of the various inventive aspects. This is however not to be interpreted as the invention requiring more features than the ones expressly recited in the independent claims. Furthermore, combinations of features of different embodiments and obvious known alternative structural means are meant to be within the scope of the present description, as would be clearly understood and derived by those skilled in the art at the time of the invention. Additionally, in some examples, well-known methods, structures and techniques have not been shown in detail in order not to obscure the conciseness of the description.

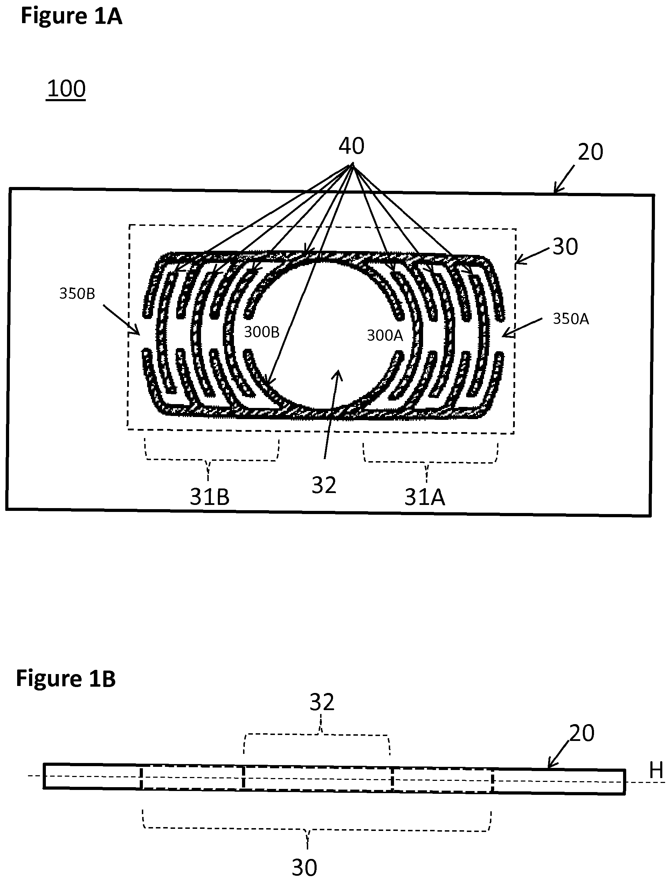

**Figure 1A** shows a top view of a general schematic representation of an electrode holding arrangement **100** according to a first exemplary embodiment. The electrode holding arrangement **100** may be for example a wrist band, a headband, a headset or any other arrangement or structure that shall hold one or more electrodes pressed towards a certain tissue surface in order to make a stable and reliable electrical connection. The electrode holding arrangement **100** may comprise a piece of flexible material **20,** such as for example a plastic material, in which at least one electrode spring element **30** is generated. The electrode spring element **30** may be created, for example, by cutting a profile **40** (indicated in the figure by a dark/diagonal pattern profile) of the piece of flexible material **20.** Such cut profile may for example define the contour of the electrode spring element **30.** According to an exemplary embodiment, the contour may be cut out of a thin plate of the piece of flexible material **20** by means of conventional cutting, laser cutting or cutting with a highpressure jet stream. According to exemplary embodiments, the electrode holding arrangement **100** may comprise just one piece of flexible material **20,** but may also comprise a number of pieces of flexible material **20** assembled together. The piece or pieces of flexible material **20** may be for example straps or plates of variable forms and with a thickness between 0.2 to 5mm. A side view of such strap or plate can be seen in **Figure 1B** in which the electrode spring element **30** is in neutral state, that is, not in operation. According to exemplary embodiments, the piece or pieces of flexible material **20** may be made of a plastic, but could also be made of cardboard or other materials with flexible characteristics and performance similar to plastic.

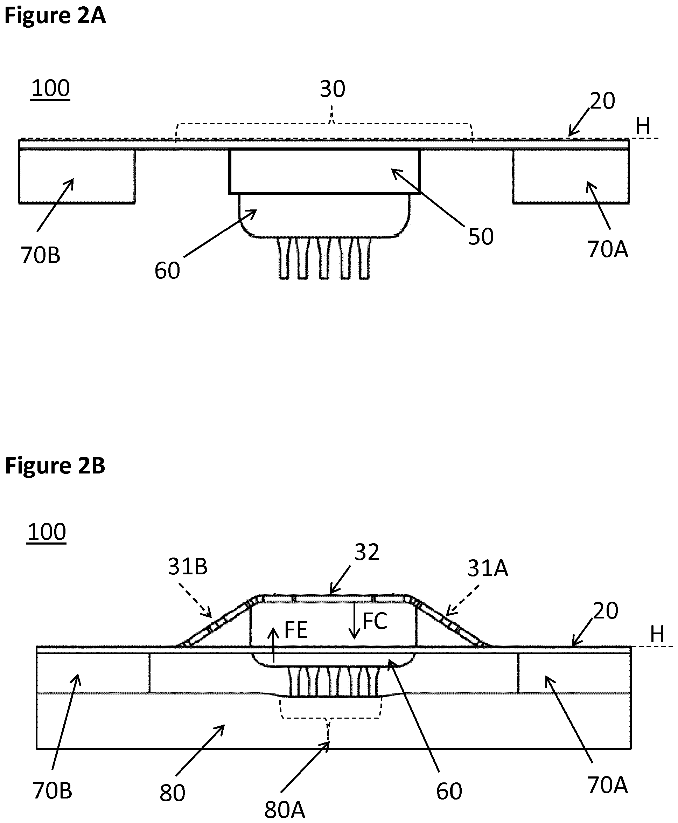

The electrode spring element **30** is therefore part of, and made from and within, the piece of flexible material **20,** e.g. by just cutting out pieces of that flexible material. According to an exemplary embodiment, the electrode spring element **30** comprises at least a first connection arrangement **31A,** a middle segment piece **32** and a second connection arrangement **31B** and its geometry is designed to provide that element with a spring property or action such that an electrode placed under the middle segment piece **32** is pressed towards the body tissue surface in that location. According to an exemplary embodiment, the first and second connection arrangements **31A** and **31B** of the electrode spring element **30** connect a different side or portion **300A** and **300B** of the middle segment piece **32** to a different anchor point **350A** and **350B** of the piece of flexible material **20** and such that, in operation (as shown in **Figure 2B**)**,** that is, when an electrode **60** is positioned under the middle segment piece **32** and over a tissue surface **80** and generates a force that makes that middle segment piece **32** protrude outside the horizontal plane **H** of the sheet of flexible material **20,** the first and second connection arrangements **31A, 31B** generate a compression force that pulls the middle segment piece **32** and hence forces the electrode towards that tissue surface. According to an exemplary embodiment the first and second connection arrangements **31A, 31B** of the electrode spring element **30** have the same geometry and connect an opposite side of the middle segment piece **32.** According to an exemplary embodiment, the first and second connection arrangements **31A, 31B** may be designed such that the compression force is perpendicular to the tissue surface. This advantageously maintains the electrode perpendicular to the tissue surface and helps improve the electrode-skin contact, which assures better electrical signal quality transfer. According to an exemplary embodiment the first and second connection arrangements **31A, 31B** comprise a plurality of extendable elements **370** in the form of connected U-shaped segments. According to exemplary embodiments the middle segment piece **32** has a circular or substantially circular shape, but other shapes are possible, like for example elliptical or orthogonal shapes.

It shall be also understood that the force by which the electrode is pressed to the tissue will depend on the flexible material characteristics and the geometry of the electrode spring element **30,** and more specifically to the implementation of the design of the first and second connection arrangements **31A** and **31B.** The number of extendable elements **370** of the first and second connection arrangements that connect the middle segment piece **32** to the piece of flexible material **20** and their form will also play an important role in achieving the desired pressure performance characteristics.

**Figure 2A** shows a side view of the electrode holding arrangement **100** of figure 1 comprising a piece of flexible material **20** and an electrode **60** attached to an electrode spring element **30** formed within the piece of flexible material. According to exemplary embodiments, the electrode **60** may be attached directly to the middle segment piece **32** of the electrode spring element **30** or attached to an electrode holder or retainer **50** which is fixed to that middle segment piece **32.** The electrode spring element **30** of the figure is in neutral position as it does not protrude outside of the horizontal plane **H** of the sheet of flexible material **20.** According to an exemplary embodiment, the electrode holding arrangement **100** may further comprise a plurality of foam layers **70A, 70B** attached to the inner side of the plastic sheet piece of flexible material **20.**

**Figure 2B** shows a side view of the electrode holding arrangement **100** of figure 1A and 2A and in which the electrode spring element **30** is in operation, that is, when the electrode **60** is positioned under the middle segment piece **32** and on the surface of a body tissue **80,** it generates a force **FE** that makes the middle segment piece **32** protrude outside the horizontal plane **H** of the plastic piece of flexible material **20,** and the first and second connection arrangements **31A, 31B** generate a compression force **FC** that pulls the middle segment piece **32** and hence the electrode **60** towards that body tissue surface location **80A.** According to an exemplary embodiment, the electrode holding arrangement **100** also comprises a plurality of foam layers **70A, 70B** attached between the inner side of the plastic sheet piece of flexible material **20** and the body tissue **80** in order to help maintain a certain distance between the electrode and the tissue surface, and to provide additional comfort to the electrode holding arrangement wearer subject.

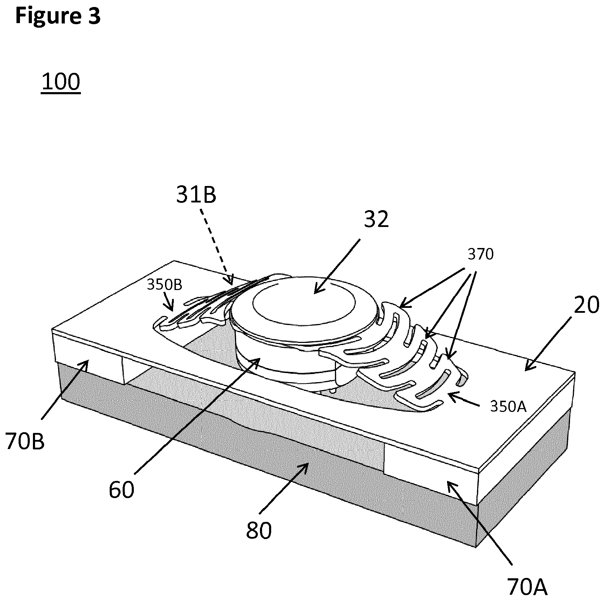

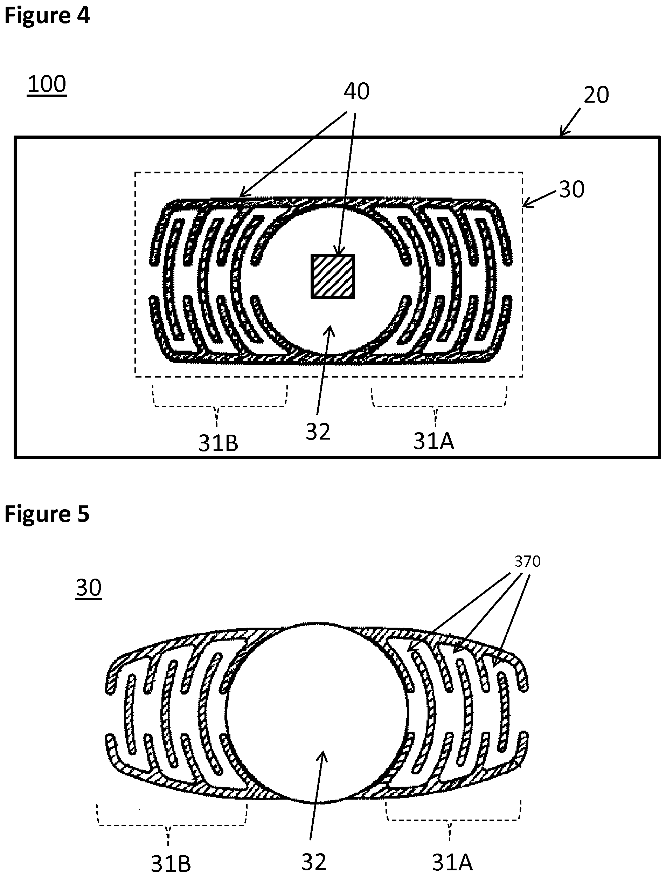

A perspective view of an electrode holding arrangement **100** according to an exemplary embodiment, when the electrode spring element **30** is in operation, is shown in **Figure 3****.** In particular, the exemplary embodiment of the electrode spring element is described in figure 5.

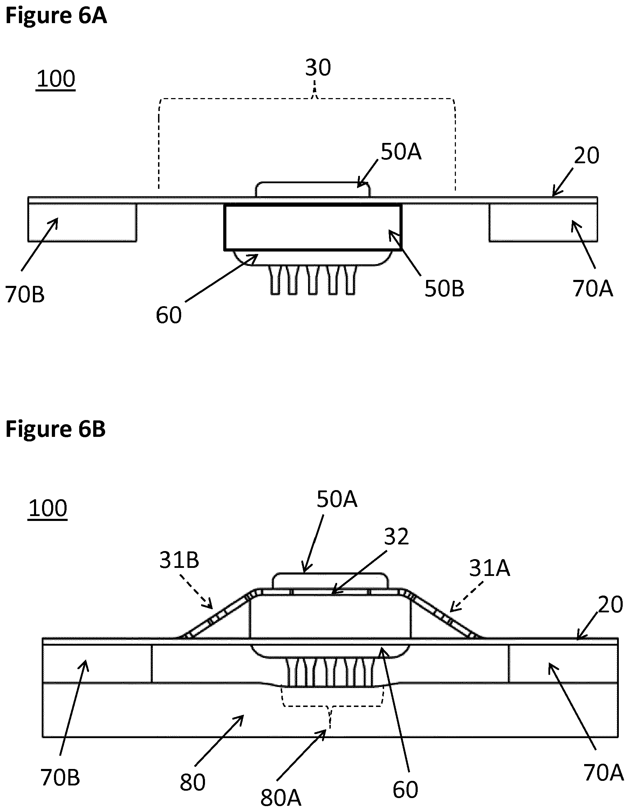

**Figure 4** shows a top view of a general schematic representation of an electrode holding arrangement **100** according to a second exemplary embodiment. The electrode holding arrangement **100** is basically the same as the one shown in figure 1A, with the difference that the cut profile **40** and hence the electrode spring element **30** provides for an opening in the middle segment piece **32.** Such opening may have variable shapes and is intended for providing further connection functionality between the middle segment piece **32** and the electrode holder or retainer **50.** For example, instead of just attaching the electrode holder or retainer **50** to one side (the inner side towards the tissue surface) of the middle segment piece **32** as shown in figure 2A, such electrode holder may comprise two holding elements **50A, 50B** connected through the opening of the middle segment piece **32** as shown in **Figure 6A****,** which provides improved connectivity between the electrode spring element **30** and the electrode **60.** **Figure 6B** shows the electrode holding arrangement **100** of figures 4 and 6A in operation, similar to what is shown in figures 2B and 3.

**Figure 5** shows a top view of another exemplary implementation of an electrode spring element **30** according to a third exemplary embodiment. In this embodiment the first and second connection arrangements **31A, 31B** comprise a plurality of extendable elements **370** in the form of connected U-shaped segments and in which the U-shaped segments do not have the same length, that is, the extendable elements vary in size, compared to the U-shaped segments of for example figure 4 which have the same length and size. According to another exemplary embodiment the middle segment piece **32** of the electrode spring element **30** may also comprise an opening as shown in figure 4.

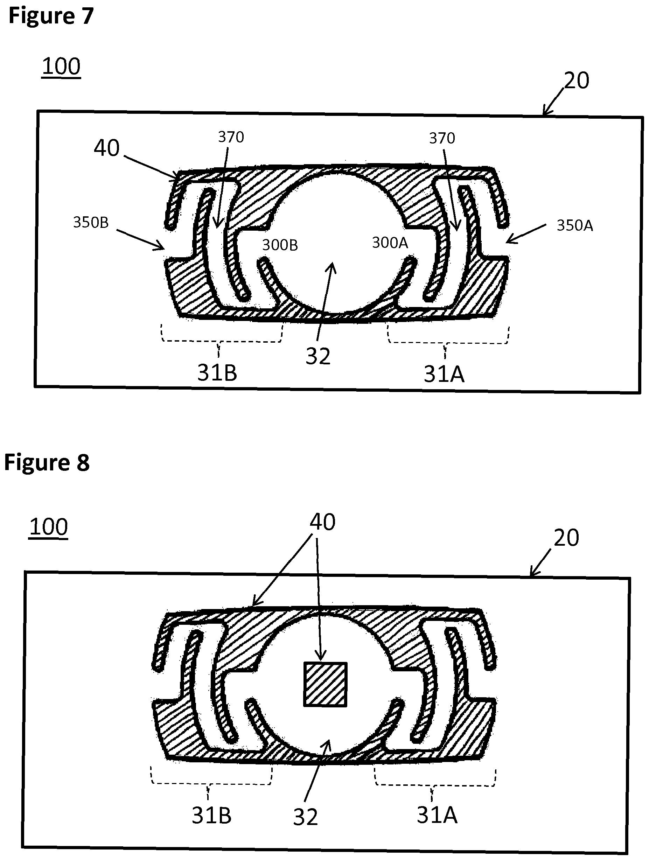

**Figure 7** shows a top view of a general schematic representation of an electrode holding arrangement **100** according to a fourth exemplary embodiment, comprising a piece of flexible material **20,** such as a plastic material, in which at least one electrode spring element is generated. The electrode spring element may be created, for example, by cutting a profile **40** (indicated in the figure by a dark/diagonal pattern profile) of the piece of flexible material **20.** According to an exemplary embodiment, the electrode spring element comprises at least a first connection arrangement **31A,** a middle segment piece **32** and a second connection arrangement **31B** and its geometry is designed to provide that element with a spring property or action such that an electrode placed under the middle segment piece **32** is pressed towards the body tissue surface in that location. According to an exemplary embodiment, the first and second connection arrangements **31A, 31B** of the electrode spring element **30** connect a different side or portion **300A** and **300B** of the middle segment piece **32** to a different anchor point **350A** and **350B** of the piece of flexible material **20.** In this exemplary embodiment, the first and second connection arrangements **31A, 31B** comprise an extendable element **370** in the form of a curved segment connection or in the form of a meander. According to exemplary embodiments the middle segment piece **32** has a circular or substantially circular shape, but other shapes are possible, like for example elliptical or orthogonal shapes. **Figure 8** shows a top view of a general schematic representation of another electrode holding arrangement **100** according to a fifth exemplary embodiment, which comprises basically the same elements as the electrode holding arrangement **100** of figure 7 but now the middle segment piece **32** of the electrode spring element **30** has also an opening for improved fixation to an electrode or electrode holder.

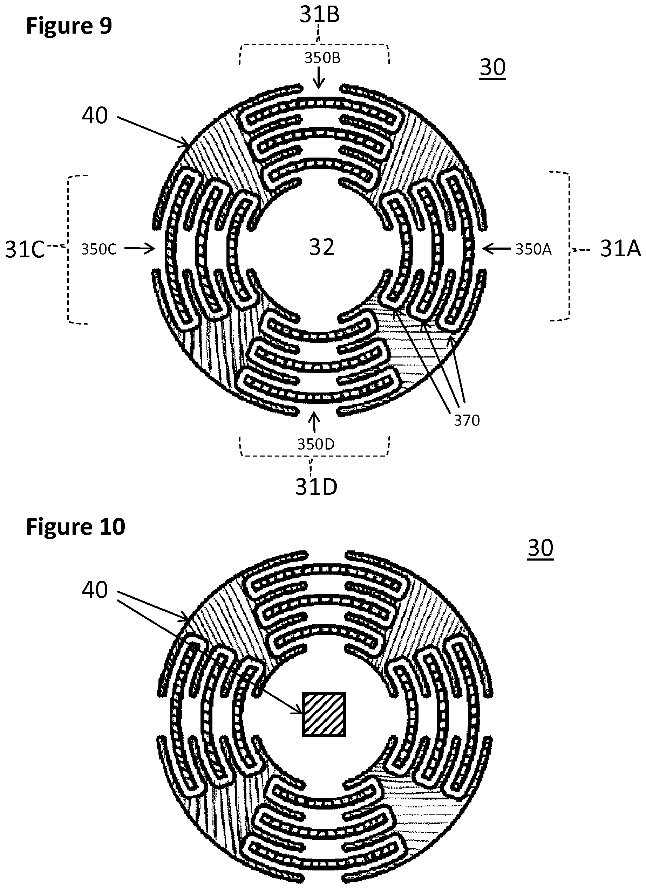

**Figure 9** shows a top view of another exemplary implementation of an electrode spring element **30** according to a sixth exemplary embodiment wherein the cut profile **40** provides for a circular shape electrode spring element geometry. In this embodiment the electrode spring element **30** comprises four connection arrangements **31A, 31B, 31C, 31D** that connect the middle segment piece **32** to different anchor points **350A, 350B, 350C, 350D** of the piece of flexible material **20** (not shown). The four connection arrangements comprise a plurality of extendable elements **370** in the form of connected U-shaped segments and in which the U-shaped segments do not have the same length or size. **Figure 10** shows basically the same electrode spring element **30** as the one shown in figure 9 but now the middle segment piece **32** of the electrode spring element **30** has also an opening for improved fixation to an electrode or electrode holder.

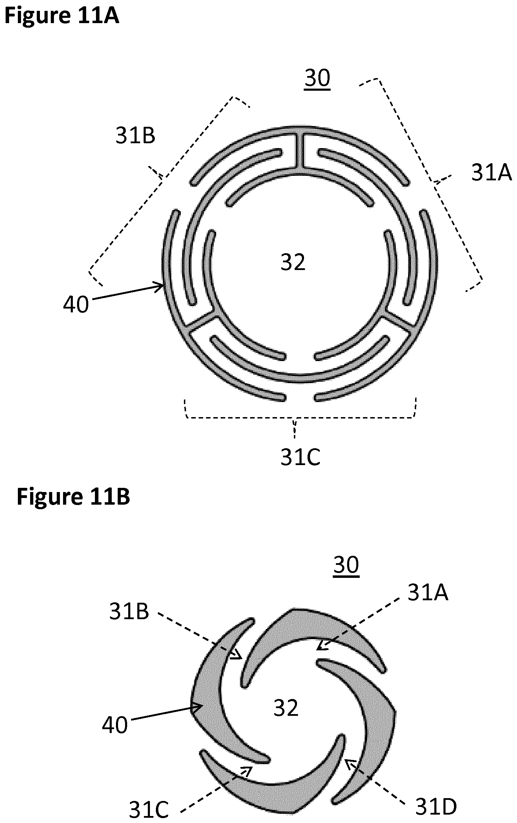

**Figure 11A** shows a top view of another exemplary implementation of an electrode spring element according to an eighth exemplary embodiment. In this embodiment the electrode spring element **30** and cut profile **40** (in this figure represented by a grey area) provide for a circular geometry and comprises three connection arrangements **31A, 31B, 31C** that connect the middle segment piece **32** to the piece of flexible material **20.** The three connection arrangements comprise one extendable element **370** in the form of connected U-shaped segments. The connection arrangements in this exemplary embodiment circumvent the middle segment piece **32** and advantageously increase stability of the electrode. **Figure 11B** shows a top view of another exemplary implementation of an electrode spring element according to a ninth exemplary embodiment. In this embodiment the electrode spring element **30** and cut profile **40** (in this figure represented by a grey area) provide for a substantially circular geometry and comprises four connection arrangements **31A, 31B, 31C, 31D** that connect the middle segment piece **32** to the piece of flexible material **20.** The four connection arrangements comprise one extendable element **370** in the form of a curved segment. According to exemplary embodiments, the middle segment piece **32** of the electrode spring elements of figures 11A and 11B may also comprise an opening as shown for example in figures 4, 8 and 10 for improved fixation to an electrode or electrode holder.

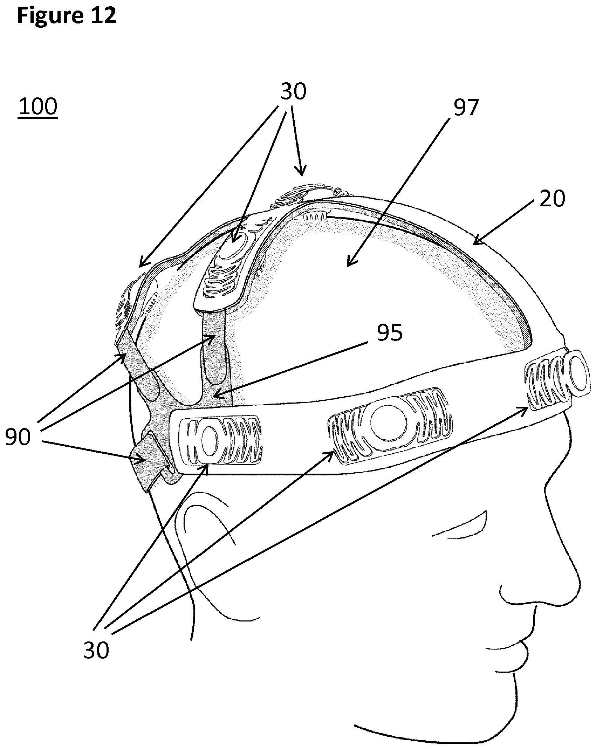

**Figure 12** shows an exemplary electrode holding arrangement **100** in the form of a headset device, showing a plurality of electrode spring elements **30** implemented in a single piece of flexible material **20.** According to an exemplary embodiment, the single piece of flexible material is also cut in a form that fits the head **97** of a subject and can be fixed and secured to the subject's head by means of fixing straps **90** and one or more fittings **95.** According to an exemplary embodiment, the cut out of the plastic sheet form and the electrode spring elements may be done at the same time and in a single step. Electrodes may be fixed to the electrode spring elements for sensing purposes. The headset may comprise a foam layer attached between the inner side of the plastic sheet piece of flexible material **20** and the scalp in order to help maintain a certain distance between the electrodes and the head surface, and to provide additional wearing comfort to the subject.

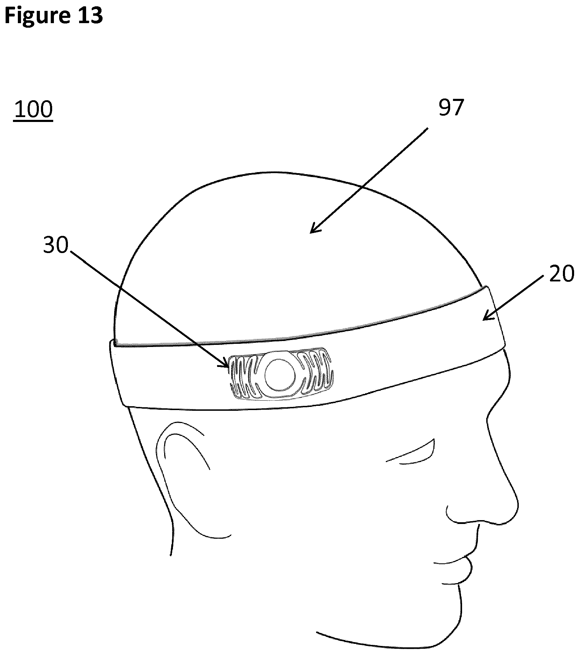

**Figure 13** shows another exemplary electrode holding arrangement **100** in the form of a headband, made of a single piece of flexible material **20,** e.g. a plastic sheet that has been cut in the form of a headband, and in which at least one electrode spring element **30** has been generated. According to an exemplary embodiment, the cut out of the plastic sheet form and the electrode spring element may be done at the same time and in a single step. An electrode may be fixed to the electrode spring element for sensing purposes. The headband may also comprise a foam layer attached between the inner side of the plastic sheet piece of flexible material and the scalp in order to help maintain a certain distance between the electrode and the skin surface, and to provide additional wearing comfort to the subject. According to an exemplary embodiment, each side of the single piece of flexible material may be connected together by known fixing or fitting means and then fitted and secured to the head **97** of the subject.

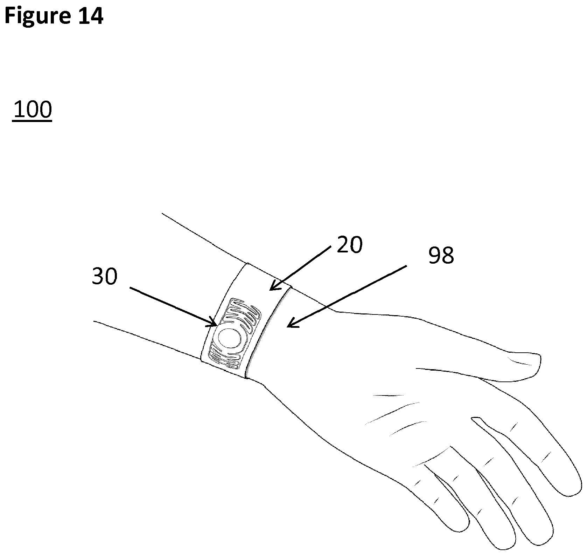

**Figure 14** shows another exemplary electrode holding arrangement **100** in the form of a wristband, made of a single piece of flexible material **20,** e.g. a plastic sheet that has been cut in the form of a wristband, and in which at least one electrode spring element **30** has been generated. According to an exemplary embodiment, the cut out of the plastic sheet form and the electrode spring element may be done at the same time and in a single step. An electrode may be fixed to the electrode spring element for sensing purposes. The wristband may also comprise a foam layer or other material attached between the inner side of the piece of flexible material and the wrist skin in order to help maintain a certain distance between the electrode and the skin surface, and to provide additional wearing comfort to the subject. According to an exemplary embodiment, each side of the single piece of flexible material may be connected together by known fixing or fitting means and then fitted and secured to the wrist **98** of the subject.

## Claims

1. An electrode holding arrangement (100) for biosignal acquisition or stimulation, comprising:
a piece of flexible material (20) adapted for being fixed to a certain body part (97, 98); and
at least one electrode spring element (30) created within and made from said piece of flexible material (20) by cutting out a certain geometric profile (40) from said flexible material (20), and configured for, in operation, when the piece of flexible material (20) is fixed to the body part (97, 98) and an electrode (60) is attached to the at least one electrode spring element (30) and is positioned on a body tissue surface (80A), the electrode spring element (30) generates a force (FC) that presses the electrode (60) against the body tissue surface (80A); wherein the electrode spring element (30) comprises at least a first connection arrangement (31A) and a second connection arrangement (31B) connected to a middle segment piece (32); and is
**characterized by**
the electrode spring element (30) is configured such that, when in operation, the electrode spring element (30) protrudes outside the plane (H) of the piece of flexible material away of the body tissue surface and the first and second connection arrangement pull the middle segment piece, and the electrode located under the middle segment piece, towards that body tissue surface location (80A).

2. An electrode holding arrangement (100) according to claim 1, wherein the at least first and second connection arrangement (31A, 31B) comprise at least one extendable element (370).

3. An electrode holding arrangement (100) according to claim 2, wherein the extendable element (370) comprises a segment with a curved or meander shape.

4. An electrode holding arrangement (100) according to claim 2, wherein the extendable element (370) comprises connected U-shaped segments.

5. An electrode holding arrangement (100) according to claim 4, wherein the connected U-shaped segments have different lengths.

6. An electrode holding arrangement (100) according to claims 3 or 4, comprising 3 or 4 connection arrangements (31A, 31B, 31C, 31D).

7. An electrode holding arrangement (100) according to any of claims 2 to 6, wherein the middle segment piece (32) has an opening for fixing an electrode (60) to the electrode spring element (30).

8. An electrode holding arrangement (100) according to any of the previous claims, wherein the electrode spring element further comprises an electrode holder (50).

9. An electrode holding arrangement (100) according to any of the previous claims, further comprising fixing and/or fitting means (90) to secure the piece of flexible material to a certain body part (97, 98).

10. Wearable device or garment comprising an electrode holding arrangement (100) according to any of the previous claims.

11. A wearable device or garment according to claim 10 and in which the electrode holding arrangement (100) is configured as a headset, headband or wristband for biosignal acquisition or stimulation.

12. A method for manufacturing an electrode holding arrangement (100), comprising:
cutting a piece of flexible material (20) into a form that fits a certain body part (97, 98);
cutting out a certain geometric profile (40) from said flexible material (20) in order to create at least one electrode spring element (30) within said piece of flexible material (20), such that the at least one electrode spring element (30) is configured for, in operation, when the piece of flexible material (20) is fixed to the body part (97, 98) and an electrode (60) is attached to the at least one electrode spring element and is positioned on a body tissue surface (80A) the electrode spring element generates a force (FC) that presses the electrode (60) against the body tissue surface (80A); wherein the electrode spring element (30) comprises at least a first connection arrangement (31A) and a second connection arrangement (31B) connected to a middle segment piece (32); and is
**characterized in that**
the electrode spring element (30) is configured such that, when in operation, the electrode spring element (30) protrudes outside the plane (H) of the piece of flexible material away of the body tissue surface and the first and second connection arrangement pull the middle segment piece, and the electrode located under the middle segment piece, towards that body tissue surface location (80A).

## Patentansprüche

1. Elektrodenhalteanordnung (100) zur Biosignalgewinnung oder Stimulation, umfassend:
ein Stück biegsames Material (20), angepasst, um an einem bestimmten Körperteil (97, 98) befestigt zu werden; und
mindestens ein Elektrodenfederelement (30), geschaffen innerhalb des Stücks und hergestellt aus dem Stück von biegsamem Material (20) durch Ausschneiden eines bestimmten geometrischen Profils (40) aus dem biegsamen Material (20), und so ausgelegt, damit bei Betrieb, wenn das Stück von biegsamem Material (20) an dem Körperteil (97, 98) befestigt ist und eine Elektrode (60) an dem mindestens einen Elektrodenfederelement (30) angebracht ist und auf einer Körpergewebeoberfläche (80A) positioniert ist, das Elektrodenfederelement (30) eine Kraft (FC) erzeugt, die die Elektrode (60) gegen die Körpergewebeoberfläche (80A) drückt; wobei das Elektrodenfederelement (30) mindestens eine erste Verbindungsanordnung (31A) und eine zweite Verbindungsanordnung (31B), verbunden mit einem mittleren Segmentstück (32), umfasst; und
**dadurch gekennzeichnet ist, dass**
das Elektrodenfederelement (30) so ausgelegt ist, dass, wenn in Betrieb, das Elektrodenfederelement (30) aus der Ebene (H) des Stücks von biegsamem Material von der Körpergewebeoberfläche wegragt und die erste und zweite Verbindungsanordnung das mittlere Segmentstück ziehen, und die Elektrode sich unter dem mittleren Segmentstück befindet, hin zu der Körpergewebeoberflächestelle (80A).

2. Elektrodenhalteanordnung (100) nach Anspruch 1, wobei die mindestens erste und zweite Verbindungsanordnung (31A, 31B) mindestens ein verlängerbares Element (370) umfassen.

3. Elektrodenhalteanordnung (100) nach Anspruch 2, wobei das verlängerbare Element (370) ein Segment mit einer gekrümmten Form oder Meanderform umfasst.

4. Elektrodenhalteanordnung (100) nach Anspruch 2, wobei das verlängerbare Element (370) verbundene U-förmige Segmente umfasst.

5. Elektrodenhalteanordnung (100) nach Anspruch 4, wobei die verbundenen U-förmigen Segmente unterschiedliche Längen aufweisen.

6. Elektrodenhalteanordnung (100) nach Ansprüchen 3 oder 4, umfassend 3 oder 4 Verbindungsanordnungen (31A, 31B, 31C, 31D).

7. Elektrodenhalteanordnung (100) nach einem der Ansprüche 2 bis 6, wobei das mittlere Segmentstück (32) eine Öffnung zur Befestigung einer Elektrode (60) an dem Elektrodenfederelement (30) aufweist.

8. Elektrodenhalteanordnung (100) nach einem der vorstehenden Ansprüche, wobei das Elektrodenfederelement weiter einen Elektrodenhalter (50) umfasst.

9. Elektrodenhalteanordnung (100) nach einem der vorstehenden Ansprüche, die weiter Befestigungs- und/oder Passungsmittel (90) zum Befestigen des Stücks von biegsamem Material an einem bestimmten Körperteil (97, 98) umfasst.

10. Tragbare Vorrichtung oder Bekleidung, umfassend eine Elektrodenhalteanordnung (100) nach einem der vorstehenden Ansprüche.

11. Tragbare Vorrichtung oder Bekleidung nach Anspruch 10 und wobei die Elektrodenhalteanordnung (100) als ein Headset, Stirnband oder Armband zur Biosignalgewinnung oder Stimulation ausgelegt ist.

12. Verfahren zur Herstellung einer Elektrodenhalteanordnung (100), umfassend:
Schneiden eines Stücks von biegsamem Material (20) zu einer Form, die mit einem bestimmten Körperteil (97, 98) passt;
Ausschneiden eines bestimmten geometrischen Profils (40) aus dem biegsamen Material (20), um mindestens ein Elektrodenfederelement (30) in dem Stück von biegsamem Material (20) zu schaffen, sodass das mindestens eine Elektrodenfederelement (30) ausgelegt ist, damit, bei Betrieb, wenn das Stück von biegsamem Material (20) an dem Körperteil (97, 98) befestigt ist und eine Elektrode (60) an dem mindestens einen Elektrodenfederelement angebracht ist und auf einer Körpergewebeoberfläche (80A) positioniert ist, das Elektrodenfederelement eine Kraft (FC) erzeugt, die die Elektrode (60) gegen die Körpergewebeoberfläche (80A) drückt; wobei das Elektrodenfederelement (30) mindestens eine erste Verbindungsanordnung (31A) und eine zweite Verbindungsanordnung (31B), verbunden mit einem mittleren Segmentstück (32), umfasst; und
**dadurch gekennzeichnet ist, dass**
das Elektrodenfederelement (30) so ausgelegt ist, dass, wenn in Betrieb, das Elektrodenfederelement (30) aus der Ebene (H) des Stücks von biegsamem Material von der Körpergewebeoberfläche wegragt und die erste und zweite Verbindungsanordnung das mittlere Segmentstück ziehen, und die Elektrode sich unter dem mittleren Segmentstück befindet, hin zu der Körpergewebeoberflächestelle (80A).

## Revendications

1. Arrangement de support d'électrode (100) pour une acquisition ou une stimulation d'un biosignal, comprenant :
une pièce d'un matériau flexible (20) adaptée à être fixée à une certaine partie corporelle (97, 98) ; et
au moins un élément de ressort d'électrode (30) créé à l'intérieur et fait à partir de ladite pièce de matériau flexible (20) en découpant un certain profil géométrique (40) à partir dudit matériau flexible (20), et configuré pour que, en fonctionnement, lorsque la pièce de matériau flexible (20) est fixée à la partie corporelle (97, 98) et qu'une électrode (60) est attachée à l'au moins un élément de ressort d'électrode (30) et est positionnée sur une surface de tissu corporel (80A), l'élément de ressort d'électrode (30) génère une force (FC) qui presse l'électrode (60) contre la surface de tissu corporel (80A) ; dans lequel l'élément de ressort d'électrode (30) comprend au moins un premier arrangement de connexion (31A) et un deuxième arrangement de connexion (31B) connecté à une pièce de segment médian (32) ; et est
**caractérisé en ce que**
l'élément de ressort d'électrode (30) est configuré de sorte que, lorsque en fonctionnement, l'élément de ressort d'électrode (30) dépasse en dehors du plan (H) de la pièce de de matériau flexible en s'éloignant de la surface de tissu corporel et le premier et le deuxième arrangements de connexion tirent la pièce de segment médian, et l'électrode localisée sous la pièce de segment médian, en direction de cette localisation de surface de tissu corporel (80A).

2. Arrangement de support d'électrode (100) selon la revendication 1, dans lequel les au moins un premier et deuxième arrangements de connexion (31A, 31B) comprennent au moins un élément extensible (370).

3. Arrangement de support d'électrode (100) selon la revendication 2, dans lequel l'élément extensible (370) comprend un segment avec une forme incurvée ou sinueuse.

4. Arrangement de support d'électrode (100) selon la revendication 2, dans lequel l'élément extensible (370) comprend des segments en forme de U connectés.

5. Arrangement de support d'électrode (100) selon la revendication 4, dans lequel les segments en forme de U connectés ont différentes longueurs.

6. Arrangement de support d'électrode (100) selon la revendication 3 ou 4, comprenant 3 ou 4 arrangements de connexion (31A, 31B, 31C, 31D).

7. Arrangement de support d'électrode (100) selon l'une quelconque des revendications 2 à 6, dans lequel la pièce de segment médian (32) a une ouverture pour fixer une électrode (60) à l'élément de ressort d'électrode (30).

8. Arrangement de support d'électrode (100) selon l'une quelconque des revendications précédentes, dans lequel l'élément de ressort d'électrode comprend en outre un support d'électrode (50).

9. Arrangement de support d'électrode (100) selon l'une quelconque des revendications précédentes, comprenant en outre des moyens de fixation et/ou d'ajustement (90) pour fixer la pièce de matériau flexible à une certaine partie corporelle (97, 98).

10. Dispositif ou vêtement portable comprenant un arrangement de support d'électrode (100) selon l'une quelconque des revendications précédentes.

11. Dispositif ou vêtement portable selon la revendication 10 et dans lequel l'arrangement de support d'électrode (100) est configuré en tant que casque, bandeau ou bracelet pour une acquisition ou une stimulation d'un biosignal.

12. Procédé de fabrication d'un arrangement de support d'électrode (100), comprenant :
découper une pièce d'un matériau flexible (20) en une forme qui s'ajuste à une certaine partie corporelle (97, 98) et
découper un certain profil géométrique (40) à partir dudit matériau flexible (20) afin de créer au moins un élément de ressort d'électrode (30) à l'intérieur de ladite pièce de matériau flexible (20), de sorte que l'au moins un élément de ressort d'électrode (30) est configuré pour que, en fonctionnement, lorsque la pièce de matériau flexible (20) est fixée à la partie corporelle (97, 98) et qu'une électrode (60) est attachée à l'au moins un élément de ressort d'électrode et est positionnée sur une surface de tissu corporel (80A) l'élément de ressort d'électrode génère une force (FC) qui presse l'électrode (60) contre la surface de tissu corporel (80A) ; dans lequel l'élément de ressort d'électrode (30) comprend au moins un premier arrangement de connexion (31A) et un deuxième arrangement de connexion (31B) connectés à une pièce de segment médian (32) ; et est
**caractérisé en ce que**
l'élément de ressort d'électrode (30) est configuré de sorte que, lorsque en fonctionnement, l'élément de ressort d'électrode (30) dépasse en dehors du plan (H) de la pièce de de matériau flexible en s'éloignant de la surface de tissu corporel et le premier et le deuxième arrangements de connexion tirent la pièce de segment médian, et l'électrode localisée sous la pièce de segment médian, en direction de cette localisation de surface de tissu corporel (80A).
